(19) Europäisches Patentamt
European Patent Office
Office européen des brevets

(11) Numéro de publication: **0 020 230**
**B1**

(12) **FASCICULE DE BREVET EUROPEEN**

(45) Date de publication du fascicule du brevet: **08.09.82**

(21) Numéro de dépôt: **80400682.3**

(22) Date de dépôt: **16.05.80**

(51) Int. Cl.³: **C 07 C 57/42,** C 07 C 57/60, C 07 C 51/377, A 61 K 31/19 // C07C59/48, C07C59/56, C07C59/84, C07C59/88, C07C51/083, C07C51/353, C07C51/367

(54) **Acides p-biphényl-4 méthyl-2 buten-3 oïques, procédé pour leur préparation, compositions pharmaceutiques les contenant et leur utilisation.**

(30) Priorité: **21.05.79 FR 7913065**

(43) Date de publication de la demande: **10.12.80 Bulletin 80/25**

(45) Mention de la délivrance du brevet: **08.09.82 Bulletin 82/36**

(84) Etats contractants désignés: **AT BE CH DE GB IT LI LU NL SE**

(56) Documents cités:
**FR-A-2 196 168**
**GB-A-1 030 756**
**US-A-4 011 339**

**Le dossier contient des informations techniques présentées postérieurement au dépôt de la demande et ne figurant pas dans le présent fascicule.**

(73) Titulaire: **PIERRE FABRE S.A., 125, rue de la Faisanderie, F-75116 Paris (FR)**

(72) Inventeur: **Cousse, Henri, La foun de los Noblos Chemin de Lastinos, F-81100 Castres (FR)**
Inventeur: **Mouzin, Gilbert, 21, rue Sainte Foy, F-81100 Castres (FR)**
Inventeur: **Rieu, Jean-Pierre, 10, rue Jean Mermoz, F-81100 Castres (FR)**
Inventeur: **Tarayre, Jean-Pierre, Rue des Sports, Valdurenque F-81100 Castres (FR)**

(74) Mandataire: **Doat, Jean Pierre et al, 17, Avenue Jean Moulin, F-81106 Castres Cedex (FR)**

**EP 0 020 230 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Acides p-biphényl-4 méthyl-2 buten-3 oiques, procédé pour leur préparation, compositions pharmaceutiques les contenant et leur utilisation

La présente invention concerne de nouveaux composés chimiques, des dérivés de l'acide para-biphényl-4 méthyl-2 buten-3 oïque, leur procédé de préparation et leur application en thérapeutique.

Ces nouveaux principes actifs possèdent des propriétés antalgiques et antiinflammatoires et sont utiles notamment comme médicaments pour le traitement des rhumatismes.

L'invention vise également les compositions pharmaceutiques contenant ces nouveaux composés et leurs sels thérapeutiquement acceptables.

Les composés chimiques objet de l'invention répondent à la formule générale (I):

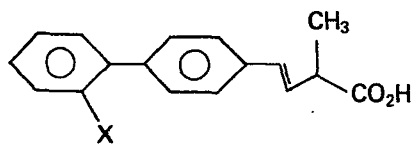

(I)

dans laquelle X représente un hydrogène ou un halogène.

Dans le brevet FR-A-2 265 409 il a été décrit des composés chimiques utilisables en thérapeutique, des céto-acides insaturés de formule générale (II):

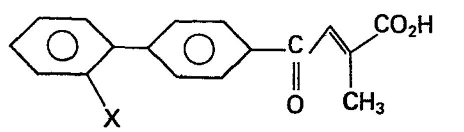

(II)

En poursuivant les investigations chimiques, à partir de ces dérivés insaturés, par réduction sélective des fonctions cétones, puis par traitement des alcools secondaires (III) par un amalgame de zinc en présence d'acide chlorhydrique concentré, il a été obtenu les nouveaux dérivés objet de la présente invention.

Les composés chimiques nouveaux suivants et leur mode de préparation sont cités à titre d'exemples non limitatifs.

Le schéma général d'accès en 4 étapes est le suivant:

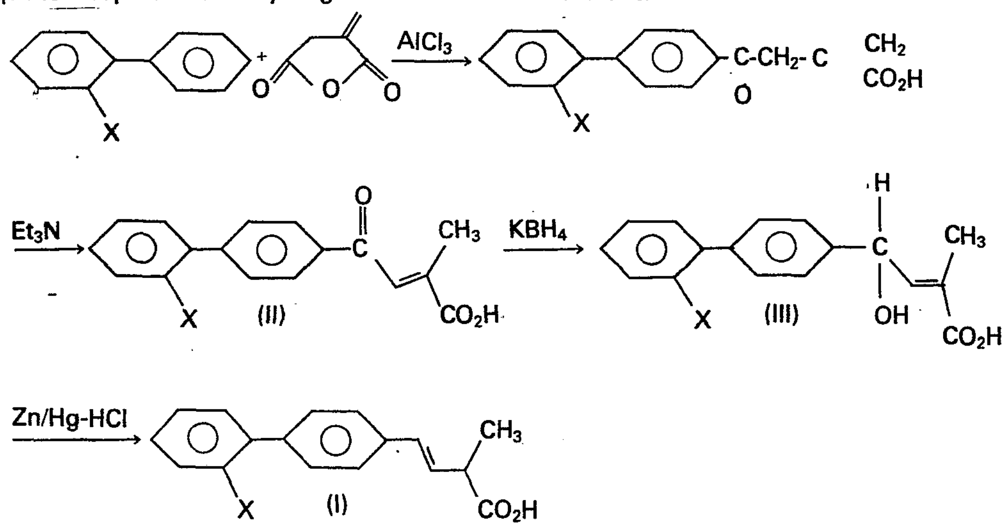


Le mécanisme de la réduction du composé (III) en composé (I) comporte la formation d'un composé intermédiaire possédant une liaison carbone-zinc métallique (intermédiaire non isolé).

Ce type de mécanisme est décrit par I. Elphimoff-Felkin et P. Sarda, Tetrahedron, *33,* 511 à 516 (1977).

1) *Préparation de l'acide trans p-biphényl-4, méthyl-2 buten-3 oïque (F 1920)*

– *1ère étape:* Préparation de l'acide (p-biphényl)-4 oxo-4 méthylène-2 butyrique (F 1377):

Une mole d'anhydride itaconique et une mole de biphényle sont dissous dans 500 $cm^3$ de nitrobenzène.

Le mélange réactionnel est refroidi à 0° C, puis l'on ajoute lentement 2,1 moles de chlorure d'aluminium en solution dans du nitrobenzène. Après quelques heures, à température ambiante, le catalyseur est détruit par une solution concentrée d'acide chlorhydrique. La phase organique est décantée, l'émulsion obtenue est traitée par de l'acétone. On récupère par filtration avec un rendement de 75% le produit de formule:

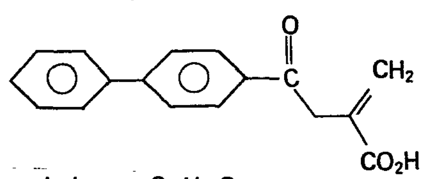

Formule brute: $C_{17}H_{14}O_3$
Masse: 266,3
Cristaux blancs
Point de fusion: 242° C
Chromatographie sur plaque:
- support: gel de silice 60 F 254 Merck
- solvant: méthanol – chloroforme, 20/80
- révélation: UV et iode
- Rf: 0,64

– *2ème étape:* Préparation de l'acide trans (p-biphényl)-4 oxo-4 méthyl-2 buten-2 oïque (F 1448):

Ajouter 12,5 ml de triéthylamine à une suspension de 5 g (16,6 moles) d'acide (p-biphényl)-4 oxo-4 méthylène-2 butyrique dans 25 ml d'eau. La solution jaune est agitée 12 heures à température ambiante, puis acidifiée par HCl (6N).

On récupère, avec un rendement de 70%, le produit de formule:

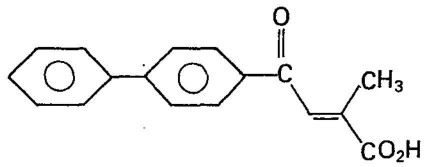

Formule brute: $C_{17}H_{14}O_3$
Masse: 266,3
Cristaux jaunes
Point de fusion: 188° C
Chromatographie sur plaque:
– support: gel de silice 60 F 254 Merck
– solvant: acide acétique – dioxane – benzène, 4/25/90
– révélation: UV et iode
– Rf: 0,61

– *3ème étape:* Préparation de l'acide trans (p-biphényl)-4 hydroxy-4 méthyl-2 buten-2 oïque (F 1546):

18 g (0,067 m) d'acide trans (p-biphényl)-4 oxo-4 méthyl-2 buten-2 oïque dans 2 litres d'eau sont neutralisés par la quantité stoechiométrique de soude à 10%; ajouter 6,75 g de borohydrure de potassium en 45 minutes. Le mélange réactionnel est laissé une nuit sous agitation à température ambiante.

Après acidification par $H_2SO_4$ à 10% en glaçant, on récupère des cristaux que l'on recristallise dans un mélange méthanol-eau 60/40.

On récupère, avec un rendement de 80% le produit de formule:

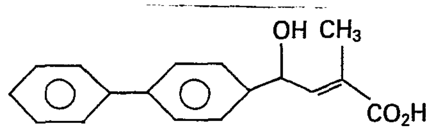

Formule brute: $C_{17}H_{16}O_3$
Masse: 268,3
Cristaux blancs
Point de fusion: 190° C
Chromatographie sur plaque:
– support: gel de silice 60 F254 Merck
– solvant: acide acétique – dioxane – benzène, 2/8/90
– révélation: UV et iode
– Rf: 0,28

– *4ème étape:* Préparation de l'acide trans p-biphényl-4 méthyl-2 buten-3 oïque (F 1920):

Dans un ballon de 250 ml, introduire successivement 21 ml d'acide chlorhydrique concentré, 9 ml d'eau distillée, 40 ml de toluène et 6 g (22,3 moles) d'acide trans p-biphényl-4 hydroxy-4 méthyl-2 buten-2 oïque.

Puis, sous agitation, introduire 12 g d'amalgame de zinc (préparé selon O.S., 56, p. 101 à 107). Le ballon est ensuite plongé dans un bain préchauffé à 120° C. Maintenir ½ heure au reflux, puis laisser revenir à température ambiante.

Décanter et extraire la phase aqueuse à l'éther éthylique:

après évaporation du solvant et recristallisation du résidu dans un mélange cyclohexane – acétate d'éthyle, on récupère avec un rendement de 65% le produit de formule:

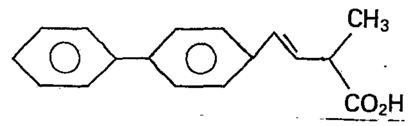

Formule brute: $C_{17}H_{16}O_2$
Masse moléculaire: 252,3
Cristaux blancs
Point de fusion: 174° C
Chromatographie sur plaque:
– support: gel de silice 60 F 254 Merck
– solvant: méthanol – chloroforme, 15/85
– révélation: UV et iode
– Rf: 0,60

2) *Préparation de l'acide trans orthochlorophényl-4' phényl-4 méthyl-2 buten-3 oïque (F 2073)*

– *1ère étape:* Préparation de l'acide [(O-chlorophényl)-4' phényl]-4 oxo-4 méthylen-2 butyrique (F 1379):

D'une façon similaire à la première étape de l'exemple 1, mais en utilisant le chlorobiphényl, on obtient, avec un rendement de 75%, le produit de formule:

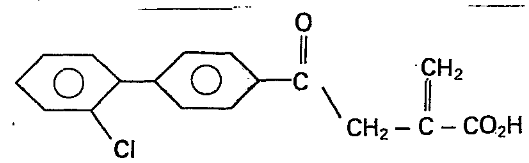

Formule brute: $C_{17}H_{13}Cl\ O_3$
Masse moléculaire: 300,7
Cristaux blancs
Point de fusion: 208° C
Chromatographie sur plaque:
– support: gel de silice 60 F 254 Merck
– solvant: acide acétique – dioxane – benzène, 4/25/90
– révélation: UV et iode
– Rf: 0,78

– *2ème étape:* Préparation de l'acide trans (orthochloro phényl-4' phényl)-4 oxo-4 méthyl'2 buten-2 oïque (F 1439):

D'une façon similaire à la deuxième étape de l'exemple 1, on obtient le produit de formule:

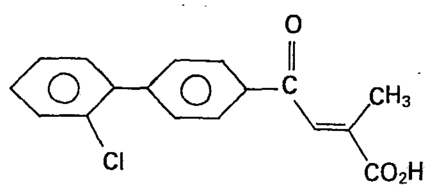

Formule brute: $C_{17}H_{13}Cl\ O_3$
Masse moléculaire: 300,7
Cristaux jaunes
Point de fusion: 196° C
Chromatographie sur plaque:
– support: gel de silice 60 F 254 Merck
– solvant: acide acétique – dioxane – benzène, 2/8/40

– révélation: UV et iode
– Rf: 0,41
– *3ème étape:* Préparation de l'acide trans (orthochloro phényl-4' phényl-4) hydroxy-4 méthyl-2 buten-2 oïque (F 1547):

D'une façon similaire à la troisième étape de l'exemple 1, on obtient le produit de formule:

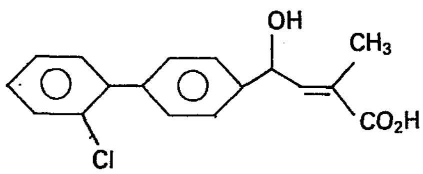

Formule brute: $C_{17}H_{15}Cl\ O_3$
Masse moléculaire: 302,7
Cristaux blancs
Point de fusion: 140° C
Chromatographie sur plaque:
– support: gel de silice 60 F 254 Merck
– solvant: acide acétique – dioxane – benzène, 2/8/90
– révélation: UV et iode
– Rf: 0,25

– *4ème étape:* Préparation de l'acide trans orthochloro phényl-4' phényl-4 méthyl-2 buten-3 oïque (F 2073):

D'une façon similaire à la quatrième étape de l'exemple 1, on obtient le produit de formule:

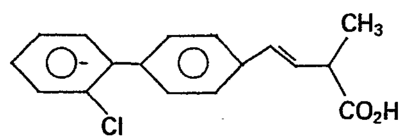

Formule brute: $C_{17}H_{15}Cl\ O_2$
Masse moléculaire: 286,75
Cristaux blancs
Point de fusion: 100°C
Chromatographie sur plaque:
– support: gel de silice 60 F 254 Merck
– solvant: méthanol – chloroforme, 15/85
– révélation: UV et iode
– Rf: 0,62

3) *Préparation de l'acide trans ortho fluoro phényl-4' phényl-4 méthyl-2 buten-3 oïque:*

D'une façon similaire à celle décrite dans l'exemple 1, dans les étapes 1 à 4, mais en utilisant l'orthofluoro biphényl, on obtient le produit de formule:

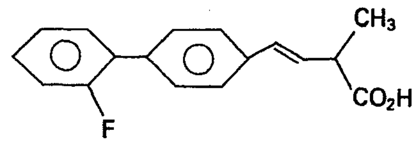

*Expérimentations:*

Les dérivés précédemment décrits ont fait l'objet d'essais pharmacologiques et toxicologiques qui ont permis de mettre en évidence d'intéressantes propriétés antalgiques.

A *Toxicologie:*

L'étude de la toxicité a été effectuée chez la souris conventionnelle pesant environ 20 grammes:

Les substances ont été administrées par voie orale, la DL 50 est calculée suivant la méthode de L.C. Miller et M.L. Tainter, Proc. Soc. Exper. Biol. Med., 1944, 57, 261.

A titre d'exemples non limitatifs, quelques résultats sont rapportés:

| Composés | DL 50 voie orale mg/kg |
|---|---|
| Acide trans p-biphényl-4 méthyl-2 buten-3 oïque | 500 |
| Acide trans orthochlorophényl-4' phényl-4 méthyl-2 buten-3 oïque | 750 |
| Acide trans orthofluorophényl-4' phényl-4 méthyl-2 buten-3 oïque | 1000 |

B *Pharmacologie:*

Les propriétés anti-inflammatoires ont été révélées par le test de l'oedème provoqué par injection de carragénine dans la patte du rat selon la technique de C. Winter, E. Risley, et G. Nuss. Proc. Soc. Exper. Biol. Med., 1962, 111, 544–547.

Les produits ont été administrés par voie orale en suspension dans le mélange tween-eau 2 heures avant l'expérimentation.

Les doses diminuant de 50%, le volume de la patte est respectivement pour le F 1920 : ED 50 = 25 mg/kg, F 2073 : ED 50 = 15 mg/kg.

Les résultats obtenus sur le granulome aux pellets de coton selon la technique de R. Meier, Schuler et Desaulles-Experientia 1950, 6, 469, permettent de mettre en évidence une réduction nette du poids du granulome à partir de 10 mg/kg pour le F 1920 et le F 2073.

Les résultats sont relevés au bout de 5 jours et aucune modification n'est observée, comparativement aux témoins, en ce qui concerne l'évolution pondérale du thymus et des capsules surrénales.

Sur la polyarthrite provoquée par l'adjuvant de Freund chez le rat selon B.B. Newbould – Brit. J. Pharmacol. Chemother. 1963, 21, 127, polyarthrite à adjuvant de Freund chez le rat, les résultats obtenus figurent dans le tableau ci-dessous et sont exprimés comparativement aux témoins pathologiques.

| Produits | Doses/jour mg/kg | Réduction de l'arthrite | | | Dosages biochimiques en fin d'expérience | |
|---|---|---|---|---|---|---|
| | | primaire | secondaire patte injectée | patte non injectée | vitesse de sédimentation | fibrinogène |
| F 1920 | 50 | – 40 | – 50 | – 48 | – 70 | – 22 |
| | 30 | – 30 | – 40 | – 38 | – 50 | – 20 |
| | 10 | – 20 | – 30 | – 20 | – 40 | – 15 |
| F 2073 | 50 | – 45 | – 60 | – 70 | – 90 | – 45 |
| | 30 | – 35 | – 50 | – 60 | – 80 | – 40 |
| | 10 | – 25 | – 40 | – 50 | – 50 | – 30 |

L'activité antalgique a été étudiée selon Siegmund et Coll. (J. Pharm. Exptl. Ther. 1957, 119, 453).

Les composés sont administrés par voie orale 30 minutes avant l'injection de phényl benzoquinone.

| Composés | DE 50 mg/kg |
|---|---|
| Acide trans p-biphényl-4 méthyl-2 buten-3 oïque | 10 |
| Acide trans orthochloro phényl-4' phényl-4 méthyl-2 buten-3 oïque | 7 |
| Acide trans orthofluoro phényl-4' phényl-4 méthyl-2 buten-3 oïque | 5 |
| Glafénine | 36 |
| Acide acétyl salicyclique | 100 |

C *Applications thérapeutiques:*

Compte tenu de la parfaite tolérance et des propriétés pharmacologiques, les composés chimiques objet de l'invention peuvent être utilisés dans le traitement d'algies rebelles à caractère inflammatoire justiciables de traitement prolongé. Les résultats se sont avérés satisfaisants dans le cas de rhumatismes inflammatoires ou dégénératifs.

Les préparations pharmaceutiques contenant ces principes actifs peuvent être administrées par voie orale, parentérale ou rectale.

La dose par unité de prise est comprise entre 50 et 200 mg.

Ces compositions pharmaceutiques peuvent également contenir d'autres principes actifs pharmaceutiquement et thérapeutiquement acceptables.

Quelques exemples de préparations pharmaceutiques contenant des principes actifs objet d'expérimentations sont donnés ci-après à titre indicatif et non limitatif.

a) *Comprimés:*

Acide trans p-biphényl-4 méthyl-2 buten-3 oïque 50 mg

Excipient: lactose

b) *Gélules:*

Acide trans ortho chloro phényl-4' phényl-4 méthyl-2 buten-3 oïque 50 mg

Méprobamate 100 mg

c) *Suppositoires* Adultes

Acide trans p-biphényl-4 méthyl-2 buten-3 oïque 100 mg

Glycérides semi-synthétiques q.s.p. 1 suppositoire de 1 g

**Revendications**

1) A titre de composés chimiques nouveaux, les dérivés de l'acide p-biphényl-4 méthyl-2 buten-3 oïque, répondant à la formule générale:

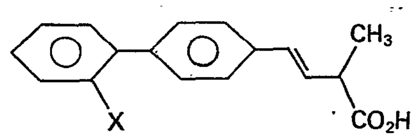

dans laquelle X peut être un hydrogène ou un halogène, ainsi que les sels inorganiques et organiques avec des bases thérapeutiquement acceptables.

2) Un composé selon la revendication 1 et plus particulièrement l'acide trans p-biphényl-4 méthyl-2 buten-3 oïque.

3) Un composé selon la revendication 1 et plus particulièrement l'acide trans ortho chloro phényl-4' phényl-4 méthyl-2 buten-3 oïque.

4) Un composé selon la revendication 1 et plus particulièrement l'acide trans orthofluorophényl-4' phényl-4 méthyl-2 buten-3 oïque.

5) Procédé de préparation des composés chimiques objet des revendications 1 à 4, caractérisé en ce que l'on traite un intermédiaire de synthèse de formule générale:

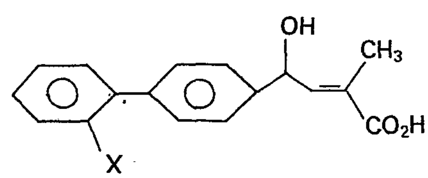

par un amalgame de zinc en présence d'acide chlorhydrique.

6) Procédé selon la revendication 5, caractérisé en ce que l'intermédiaire de synthèse est l'acide trans p-biphényl-4 hydroxy-4 méthyl-2 buten-2 oïque.

7) Procédé selon la revendication 5, caractérisé en ce que l'intermédiaire de synthèse est l'acide trans orthochloro phényl-4' phényl-4 hydroxy-4 méthyl-2 buten-2 oïque.

8) Procédé selon la revendication 5, caractérisé en ce que l'intermédiaire de synthèse est l'acide trans orthofluoro phényl-4' phényl-4 hydroxy-4 méthyl-2 buten-2 oïque.

9) Les produits selon les revendications 1 à 4 utilisables dans le traitement des troubles des rhumatismes et des algies diverses.

10) Les compositions pharmaceutiques contenant comme principe acitif au moins un produit selon l'une des revendications 1 à 4.

11) Les compositions pharmaceutiques selon la revendication 10, administrables par voie orale, rectale ou parentérale.

12) Les compositions pharmaceutiques selon l'une des revendications 10 et 11 pouvant être associées à d'autes principes actifs.

**Patentansprüche**

1. Als neue chemische Verbindungen, die Derivate der 4-p-Biphenyl-2-methyl-3-butensäure der allgemeinen Formel

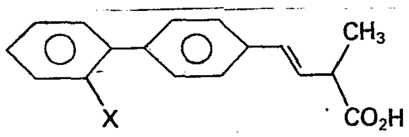

worin X ein Wasserstoffatom oder ein Halogenatom bedeutet, sowie deren anorganische und organische Säuren mit therapeutisch annehmbaren Basen.

2. Verbindung nach Anspruch 1, nämlich trans-4-p-Biphenyl-2-methyl-3-butensäure.

3. Verbindung nach Anspruch 1, nämlich trans-4-[4'-(o-Chlorphenyl)-phenyl]-2-methyl-3-butensäure.

4. Verbindung nach Anspruch 1, nämlich trans-4-[4'-(o-Fluorphenyl)-phenyl]-2-methyl-3-butensäure.

5. Verfahren zur Herstellung der chemischen Verbindungen nach den Ansprüchen 1 bis 4, dadurch gekennzeichnet, dass man ein Synthesezwischenprodukt der allgemeinen Formel

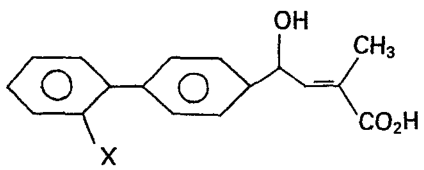

mit einem Zinkamalgam in Gegenwart von Chlorwasserstoffsäure behandelt.

6. Verfahren nach Anspruch 5, dadurch gekennzeichnet, dass man als Synthesezwischenprodukt die trans-4-[4'-(o-Chlorphenyl)-phenyl]-4-hydroxy-2-methyl-2-butensäure verwendet.

8. Verfahren nach Anspruch 5, dadurch gekennzeichnet, dass man als Synthesezwischenprodukt die trans-4-[4'-(o-Fluorphenyl)-phenyl]-4-hydroxy-2-methyl-2-butensäure verwendet.

9. Die Produkte nach den Ansprüchen 1 bis 4 zur Verwendung bei der Behandlung von rheumatischen Störungen und verschiedenartigen Schmerzen.

10. Pharmazeutische Zubereitungen, dadurch gekennzeichnet, dass sie als Wirkstoff mindestens ein Produkt gemäss einem der Ansprüche 1 bis 4 enthalten.

11. Pharmazeutische Zubereitungen nach Anspruch 10, dadurch gekennzeichnet, dass sie auf oralem, rektalem oder parenteralem Wege verabreicht werden können.

12. Pharmazeutische Zubereitungen nach einem der Ansprüche 10 und 11, dadurch gekennzeichnet, dass sie mit anderen Wirkstoffen kombiniert werden können.

**Claims**

1. As novel chemical compounds, the derivates of 4-(p-biphenyl)-2-methyl-3-butenoic acid corresponding to the general formula:

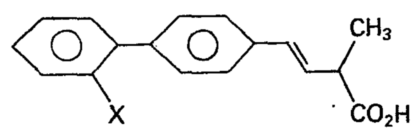

in which X may be a hydrogen or a halogen, and also the inorganic and organic salts with therapeutically acceptable bases.

2. A compound according to claim 1 and more particularly trans-4-(p-biphenyl)-2-methyl-3-butenoic acid.

3. A compound according to claim 1 and more particularly trans-4'-(o-chlorophenyl)-4-phenyl-2-methyl-3-butenoic acid.

4. A compound according to claim 1 and more particularly trans-4'-(o-fluorphenyl)-4-phenyl-2-methyl-3-butenoic acid.

5. Process for the preparation of the chemical compounds forming the subject of claims 1 to 4, characterized in that a synthesis intermediate of the generale formula:

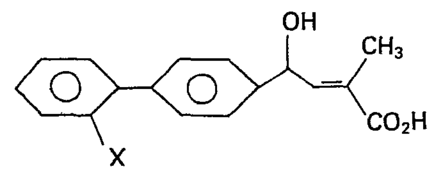

is treated with a zinc amalgam in the presence of hydrochloric acid.

6. Process according to claim 5, characterized in that the synthesis intermediate is trans-4-(p-biphenyl)-4-hydroxy-2-methyl-2-butenoic acid.

7. Process according to claim 5, characterized in that the synthesis intermediate is trans-4'-(o-chlorophenyl)-4-phenyl-4-hydroxy-2-methyl-2-butenoic acid.

8. Process according to claim 5, characterized in that the synthesis intermediate is trans-4'-(o-fluorophenyl)-4-phenyl-4-hydroxy-2-methyl-2-butenoic acid.

9. The products according to claims 1 to 4 which can be used in the treatment of rheumatic disorders and various painful conditions.

10. Pharmaceutical compositions containing as active ingredient at least one product according to one of claims 1 to 4.

11. The pharmaceutical compositions according to claim 10 which can be administered orally, rectally or parenterally.

12. The pharmaceutical compositions according to one of claims 10 and 11 which can be associated with other active ingredients.